# EUROPEAN PATENT APPLICATION

(11) **EP 1 459 775 A1**
(43) Date of publication of application: **22.09.2004**
(21) Application number: 02791990.1
(22) Date of filing: 25.12.2002
(51) Int. Cl.: A61M 5/28

(54) **SYRINGE**

(30) Priority: 28.12.2001 JP 2001401905
(71) Applicant: TERUMO KABUSHIKI KAISHA, Tokyo 151-0072 (JP)
(72) Inventor: TACHIKAWA, Kouichi, c/o Terumo kabushiki kaisha, Yamanashi 409-3853 (JP); KASAI, Masaaki, c/o Terumo kabushiki kaisha, Yamanashi 409-3853 (JP)
(74) Representative: Leson, Thomas Johannes Alois, Dipl.-Ing.
(86) International application number: PCT/JP2002/013531
(87) International publication number: WO 2003/057284

(57) **Abstract**

A syringe (1) of pre-filled type having chemicals (100) stored therein beforehand, comprising an outer tube (2), a gasket (3) slidable in the outer tube (2), a pusher (4) for movably operating the gasket (3) in the longitudinal direction of the outer tube (2), a sealing film (5) for sealing the base end opening of the outer tube (2), and a support member (6) for supporting the pusher (4) generally co-axially with the outer tube (2) on the base end side of the sealing film (5), wherein a rupture part (44) formed of a sharp projection is provided at the tip part of the pusher (4), the pusher (4) is locked to the support member (6) before use and, when the pusher (4) is rotated in a specified direction, the pusher (4) is unlocked while moving in the tip end direction thereof and, upon the unlocking, the rupture part (44) pierces the sealing film (5) to rupture and unseal the sealing film, whereby an operation to peel off and remove the sealing film for sealing the base end opening of the outer tube can be eliminated, an excellent operability can be provided, and the pusher can be prevented from being lost.

## Description

### Technical Field

The present invention relates to a syringe.

### Background Art

A syring comprise an outer tube, a gasket inserted in the outer tube, and a pusher (plunger rod) for operating the gasket to move.

Among such syringes, there have been known those which are provided with a sealing film for sealing the base end opening of the outer tube, in order to maintain the decompressed state and/or sterilized state inside the outer tube before use.

When using such a syringe, the following operations are required. First, the sealing film is peeled off and removed. A pusher appended separately is then connected to the gasket contained in the outer tube.

In such a syringe, however, it is necessary to peel off and remove the sealing film by hand, as above-mentioned, leading to troublesome operations. In addition, since the pusher is appended separately from the outer tube, there is a possibility that the pusher might be lost.

### Disclosure of Invention

It is an object of the present invention to provide a syringe whereby the need for an operation to peel off and remove a sealing film for sealing the base end opening of an outer tube can be eliminated, an excellent operability can be provided, and the pusher can be prevented from being lost.

The above object can be attained by the present invention as set forth in the following paragraphs (1) to (17).
(1) A syringe comprising:
   an outer tube;
   a gasket slidable in the outer tube;
   a sealing film for sealing the base end opening of the outer tube;
   a pusher for operating the gasket to move in the longitudinal direction of the outer tube, the pusher being provided at a tip end portion thereof with a rupture portion for rupturing the sealing film; and
   a support member for supporting the pusher generally coaxially with the outer tube on the base end side of the sealing film; wherein
   the pusher is supported by the support member before use, and at the time of use, the pusher is moved in the tip end direction relative to the outer tube, thereby causing the rupture portion to rupture and unseal the sealing film.
(2) A syringe as set forth in the above paragraph (1), wherein the rupture portion is in the shape of a sharp projection.
(3) A syringe as set forth in the above paragraph (1) or (2), comprising a lock mechanism for locking the pusher to the support member before use.
(4) A syringe as set forth in the above paragraph (3), wherein the lock mechanism is unlocked by rotating the pusher.
(5) A syringe as set forth in any of the above paragraphs (1) to (4), comprising leading unit for leading the pusher to move in the tip end direction relative to the outer tube by rotating the pusher in the state before use, wherein the movement causes the rupture portion to rupture the sealing film.
(6) A syringe as set forth in any of the above paragraphs (1) to (5), wherein the pusher comprises a connection portion for connection with the gasket so that the pusher is connectable with the gasket.
(7) A syringe as set forth in the above paragraph (6), wherein the pusher and the gasket are connectable with each other through screw-engagement between a male screw formed on the connection portion and a female screw formed in the gasket.
(8) A syringe as set forth in the above paragraph (6) or (7), wherein the rupture portion is provided at the tip end of the connection portion.
(9) A syringe as set forth in any of the above paragraphs (6) to (8), wherein the gasket and the pusher are connected with each other by an operation in the same direction as the operation to rupture and unseal the sealing film.
(10) A syringe as set forth in any of the above paragraphs (1) to (9), wherein the inside of said outer tube is in a decompressed state before use.
(11) A syringe as set forth in any of the above paragraphs (1) to (10), wherein the inside of said outer tube is in a sterilized state before use.
(12) A syringe as set forth in any of the above paragraphs (1) to (11), wherein a medical agent is preliminarily contained in the space defined by the outer tube and the gasket.
(13) A syringe as set forth in any of the above paragraphs (1) to (12), wherein the gasket comprises a recessed portion in which the rupture portion is stored.
(14) A syringe as set forth in any of the above paragraphs (1) to (13), wherein a relief portion into which at least a part of the sealing film ruptured is relieved is formed in the vicinity of the base end opening of the outer tube.
(15) A syringe as set forth in any of the above paragraphs (1) to (14), wherein the pusher comprises a rupture aid portion for cutting the sealing film in the vicinity of an edge portion of the base end opening.
(16) A syringe as set forth in any of the above paragraphs (1) to (15), wherein the pusher comprises an extension-contraction mechanism for making the whole length thereof extensible and contractible.
(17) A syringe as set forth in any of the above paragraphs (1) to (16), wherein a movement preventive member for preventing the pusher from moving in the tip end direction is removably provided on the base end side of the sealing film.

As will be described later, according to the present invention, the need for an operation to peel off and remove the sealing film for sealing the base end opening of the outer tube is eliminated. Therefore, the operation at the time of using the syringe can be performed easily and swiftly.

In addition, before use, the pusher is supported by the support member and combined with the outer tube, whereby the pusher is prevented from being lost, so that the need to search for the pusher before use is eliminated.

Besides, in the case where the lock mechanism for locking the pusher to the support member before use and the movement preventive member for preventing the pusher from moving in the tip end direction before use are provided, the rupture portion can be prevented from erroneously rupturing and unsealing the sealing film during transportation or storage.

### Brief Description of Drawings

FIG. 1 is a vertical sectional view, in a disassembled state, of a first embodiment of the syringe according to the present invention.
FIG. 2 is a vertical sectional view, in an assembled state (before use), of the first embodiment of the syringe according to the present invention.
FIG. 3 is a vertical sectional view, in the state of being used, of the syringe shown in FIGS. 1 and 2.
FIG. 4 is a perspective view of a support member in the syringe shown in FIGS. 1 and 2.
FIG. 5 is a perspective view of the support member in the syringe shown in FIGS. 1 and 2.
FIG. 6 is a partly vertical sectional view (in a contracted state) of a pusher in a second embodiment of the syringe according to the present invention.
FIG. 7 is a partly vertical sectional view (in an extended state) of the pusher in the second embodiment of the syringe according to the present invention.
FIG. 8 is a perspective view of a support member in a third embodiment of the syringe according to the present invention.
FIG. 9 is a vertical sectional view of portions in the vicinity of the support member in the third embodiment of the syringe according to the present invention.

### Best Mode for Carrying Out the Invention

Now, the syringe according to the present invention will be described in detail below, based on preferred embodiments shown in the accompanying drawings.

### <First Embodiment>

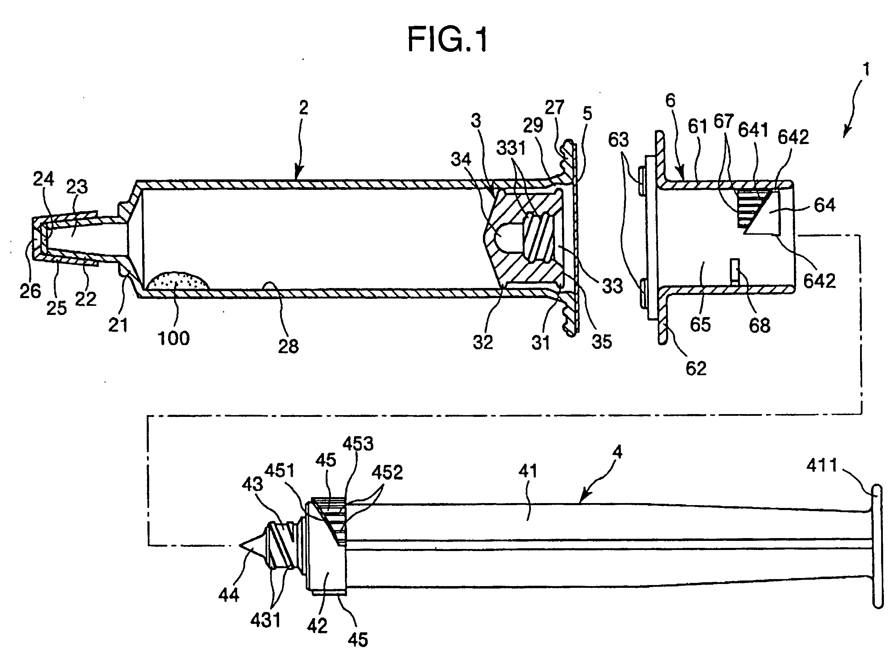

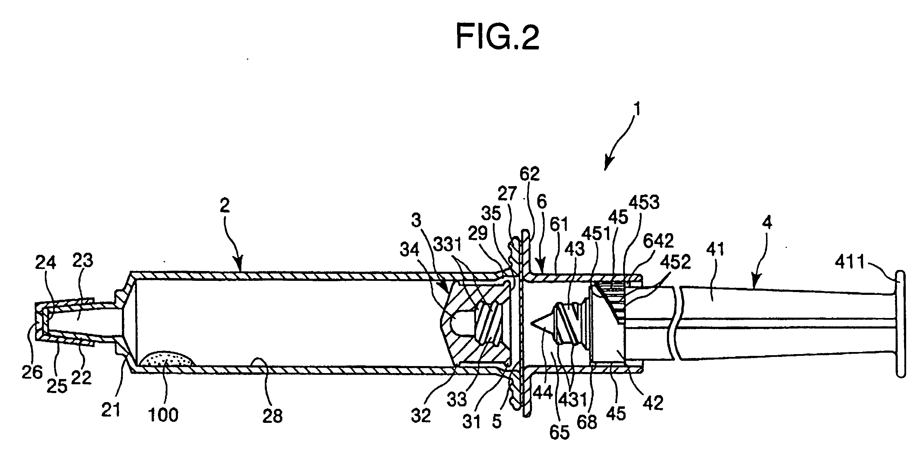

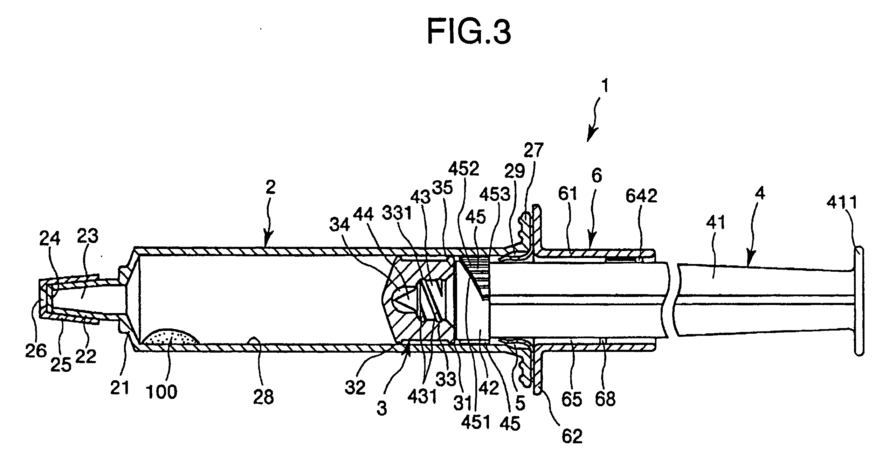

FIGS. 1 and 2 respectively are vertical sectional views, in a disassembled state and in an assembled state (before use), of a first embodiment of the syringe according to the present invention, FIG. 3 is a vertical sectional view, in the state of being used, of the syringe shown in FIGS. 1 and 2, and FIGS. 4 and 5 respectively are perspective views of a support member in the syringe shown in FIGS. 1 and 2. Incidentally, for convenience of description, the left side in FIGS. 1 to 3 will be referred to as "the tip end," and the right side as "the base end."

The syringe 1 according to this embodiment is a pre-filled syringe in which a medical agent is preliminarily contained in the inside of the syringe, and includes an outer tube (syringe outer tube) 2, a gasket 3 slidable in the outer tube 2, a pusher (plunger rod) 4 for operating the gasket 3 to move in the longitudinal direction of the outer tube 2, a sealing film 5 for sealing the base end opening of the outer tube 2, and a support member 6 for supporting the pusher 4 generally coaxially with the outer tube 2 on the base end side of the sealing film 5.

The outer tube 2 is composed of a bottomed tubular member having a bottom portion 21 on the tip end side, and a reduced diameter portion 22 reduced in diameter relative to a barrel portion of the outer tube 2 is integrally formed at a central portion of the bottom portion 21. The reduced diameter portion 22 constitutes a mouth portion through which a liquid can be let in and out.

The reduced portion 22 may be provided with a male screw (lure lock screw) at the outer circumference of a base end portion thereof.

A film 24 formed of an elastic material, as a sealing member, is mounted to the tip end of the reduced diameter portion 22, thereby sealing gas-tight the lumen 23 of the reduced diameter portion 22.

In addition, a cap 25 is fitted over and fixed to the outside of the reduced diameter portion 22. The cap 25 is provided with an opening 26 at the tip end thereof, and an outer circumferential portion of the film 24 is clamped between an edge portion of the opening 26 and the tip end face of the reduced diameter portion 22, whereby the film 24 is fixed gas-tight (liquid-tight).

Incidentally, the reduced diameter portion 22, the film 24, and the cap 25 may be adhered to each other with an adhesive or fused to each other.

The film 24 can be pierced through by a needle body such as a double ended needle. In this case, the form of the film 24 is not limited to the film form but may be a block form (plug body), as long as the film 24 can be pierced through by a needle body.

As the material for constituting the film 24, for example, those materials which will be mentioned later as the constituent material of the gasket 3 can be used.

The outer tube 2 is integrally provided with a plate-like flange 27 along the outer circumference of the base end thereof. At the time of operating the pusher 4 to move relatively to the outer tube 2 or the like, the operation can be performed by putting a finger on the flange 27.

Besides, the outer tube 2 is provided, in the vicinity of the base end opening thereof, with a relief portion 29 into which at least a part of the sealing film 5 can be relieved when the sealing film 5 is ruptured as will be described later. In the configuration shown in the figures, the relief portion 29 is constituted of an annular recessed portion (enlarged diameter portion) formed along the entire inner circumference of a base end portion of the outer tube 2.

Examples of the material usable for constituting the outer tube 2 include various resins such as polyvinyl chloride, polyethylene, polypropylene, cyclic polyolefins, polystyrene, poly-(4-methylpentene-1), polycarbonate, acrylic resin, acrylnitrile-butadiene-styrene copolymer, polyesters (e.g., polyethylene terephthalate, polyethylene naphthalate), butadiene-styrene copolymer, and polyamides (e.g., nylon 6, nylon 6 · 6, nylon 6 · 10, and nylon 12). Among these, preferred are such resins as polypropylene, cyclic polyolefins, polyesters and poly-(9-methylpentene-1), in view of their easy-moldability.

Incidentally, it is preferable for the constituent material of the outer tube 2 to be substantially transparent, in order to secure visibility of the inside of the outer tube 2.

In the outer tube 2 is contained the gasket 3 which is formed of an elastic material. An outer circumferential portion of the gasket 3 is provided with a plurality of annular projected portions 31 and 32 along the entire circumference. With the projected portions 31 and 32 slid while making close contact with the inner circumferential surface 28 of the outer tube 2, the gas-tightness (liquid-tightness) can be maintained more securely, and enhancement of slidability can be contrived.

In this embodiment, two projected portions 31 and 32 are provided along the longitudinal direction of the gasket 3. Specifically, the projected portions 31 and 32 are provided respectively on a base end portion and a tip end portion of the gasket 3.

Incidentally, in the present invention, the locations, the number, the sectional shape and the like of the projected portions 31, 32 are not limited to the above-mentioned.

In addition, the gasket 3 is provided with a hollow portion 33 opening to the base end face thereof. A connection portion 43 of the pusher 4 which will be described later is inserted (screw-engaged) in the hollow portion 33. The hollow portion 33 is provided with a female screw 331 at the inside surface thereof.

On the tip end side of the hollow portion 33, further, a recessed portion (hollow portion) 34 is formed continuously with the hollow portion 33. A rupture portion 44 of the pusher 4 which will be described later is stored in the recessed portion 34, with the gasket 3 and the pusher 4 connected with each other.

The material for constituting the gasket 3 is not particularly limited. Examples of the constituent material usable for the gasket 3 include elastic materials such as various rubber materials, e.g., natural rubber, butyl rubber, isoprene rubber, butadiene rubber, styrenebutadiene rubber, silicone rubber, etc., various thermoplastic elastomer based on polyurethane, polyester, polyamide, olefin, styrene or the like, and mixtures thereof.

Incidentally, it suffices that at least an outer circumferential portion of the gasket 3 is constituted of such an elastic material as above-mentioned. For example, a configuration in which the gasket 3 comprises a core portion (not shown) formed of a resin material and the elastic material is so disposed as to cover the outer circumference of the core portion may be adopted. In this case, the core portion is provided with the female screw 331.

As shown in FIG. 2, the gasket 3 is preferably located in the vicinity of the base end portion of the outer tube 2 before the use of the syringe 1.

In the syringe 1, a medical agent 100 is preliminarily contained in the space defined by the outer tube 2 and the gasket 3.

The medical agent 100 may be liquid or solid, but it is preferably a solid, particularly in a powdery (granular) form, and more preferably a freeze-dried product. Besides, the powdery medical agent may have been solidified into tablets, for example.

Specific examples of the medical agent 100 include vitamin agents (general vitamin agents), various amino acids, antithrombotic agents such as heparin, insulin, antibiotics, antitumor agents, analgesics, cardiacs, phleboclysis anesthetics, antiparkinsonism agents, ulcer remedies, adrenocortical hormone agents, arrhythmia remedies, correcting electrolytes, protease inhibitors, and inhibitors of tromboxane synthesis. Incidentally, it is natural that, in the present invention, the medical agent 100 is not limited to the examples just mentioned.

At a base end portion of the outer tube 2 is provided the sealing film 5 for sealing the base end opening of the outer tube 2. The sealing film 5 is adhered to the base end face of the flange 27 of the outer tube 2 along the entire circumference, by a method such as adhesion (adhesion with an adhesive or a solvent) and fusion bonding (heat fusion bonding, high-frequency fusion bonding, ultrasonic fusion bonding, or the like).

In the syringe 1, the provision of the sealing film 5 prevents the outside air from penetrating inside the outer tube 2, so that a sterilized state inside the outer tube 2 can be maintained until use. Besides, the inside of the outer tube 2 may be in a decompressed state; in this case, the presence of the sealing film 5 maintains the decompressed state inside the outer tube 2 until use.

Examples of the sealing film 5 include resin films of polyethylene, polypropylene, ionomers, polyethylene terephthalate, polystyrene, poly-(4-methylpentene-1), polyimides, etc., these resin films with an inorganic material such as silica, alumina or a mixture thereof vapor-deposited thereon, single layers of metallic foil such as aluminum foil, and laminates obtained by laminating two or more layers of these materials. Incidentally, when a laminate of a metallic foil and a resin layer having a particularly low gas permeability is used as the sealing film 5, the gas-tightness inside the outer tube 2 is enhanced, so that the sterilized state and/or the decompressed state inside the outer tube 2 can be maintained more securely.

As shown in FIG. 2, the support member 6 for supporting the pusher 4 is disposed on the base end side of the outer tube 2.

As shown in FIG. 1, the support member 6 comprises a hollow cylindrical portion 61 of a roughly circular tubular form, and a plate-like flange 62 formed integrally with the outer circumference of the tip end of the hollow cylindrical portion 61.

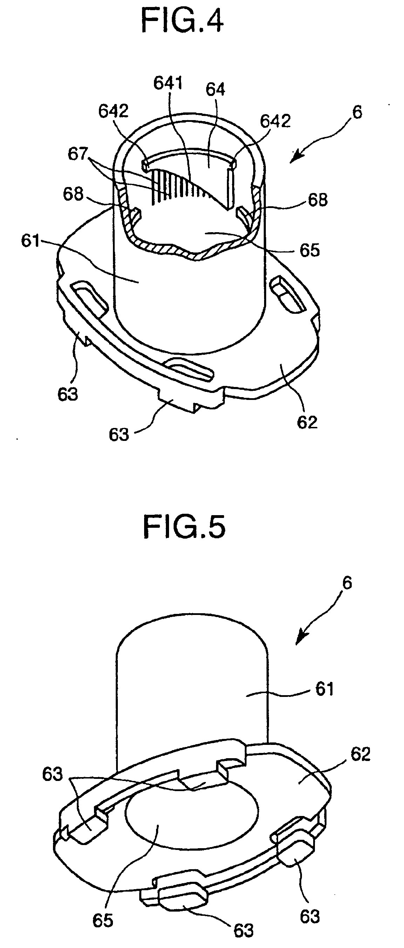

As shown in FIG. 5, at the outer circumferential portion of the flange 62 of the support member 6, there are formed a plurality of (in the configuration shown, four) claw portions (locking portions) 63 for connecting (fixing) the support member 6 to the outer tube 2. As shown in FIG. 2, in the assembled state of the syringe 1, the support member 6 and the outer tube 2 are connected (fixed) by locking the claw portions 63 to an outer edge portion of the flange 27 of the outer tube 2. In this connected condition, the hollow cylindrical portion 61 is located generally coaxially with the outer tube 2.

As shown in FIGS. 1 and 4, on the base end side of the inner circumferential surface 65 of the hollow cylindrical portion 61 is provided a protuberant portion 64 which is protuberant in a stepped manner in a roughly triangular (right-angled triangular) region. The protuberant portion 64 is formed over a range of a central angle of about 90° relative to the center axis of the hollow cylindrical portion 61. A tip end side edge portion (tip end face) of the protuberant portion 64 forms a lead surface 641 inclined relative to the circumferential direction of the hollow cylindrical portion 61.

On the tip end side of the protuberant portion 64 in the inner circumferential surface 65, there is formed a plurality of minute ribs 67 extending in the axial direction.

In addition, on the inner circumferential surface 65, a rib 68 extending in the circumferential direction is formed over a range of a central angle of about 90°, adjacently to the protuberant portion 64.

The protuberant portions 64, the ribs 67 and the ribs 68 are formed in respective pairs at symmetrical positions with respect to the center axis of the hollow cylindrical portion 61 (at positions opposite (180°) to each other).

As shown in FIG. 2, in the state before use of the syringe 1, the pusher 4 is supported on the support member 6 generally coaxially with the outer tube 2 on the base end side of the sealing film 5.

The pusher 4 comprises a main body portion 41 in such a shape that plate pieces intersect in a cross form, and a flange-formed finger receiving portion 411 is formed at the base end of the main body portion 41. In use of the syringe 1 as shown in FIG. 3, the finger receiving portion 411 is pushed by a finger or the like, thereby operating the pusher 4 to move in the tip end direction.

On the tip end side of the main body portion 41, there is provided a hollow cylindrical portion 42 having a roughly circularly tubular shape (cylindrical shape). The hollow cylindrical portion 42 is provided at its outer circumferential surface with a protuberant portion 45 which is protuberant in a stepped manner in a roughly triangular (right-angled triangular) region. The protuberant portion 45 is formed over a range of a central angle of about 90° with respect to the center axis of the hollow cylindrical portion 42. In addition, the tip end face (tip end side edge portion) 451 of the protuberant portion 45 has a portion inclined relative to the circumferential direction of the hollow cylindrical portion 42.

Such protuberant portions 45 are formed in pair at symmetrical positions (at positions opposite (180°) to each other) with respect to the center axis of the hollow cylindrical portion 42.

As shown in FIG. 2, the pusher 4 is supported in the condition where the hollow cylindrical portion 42 is inserted inside the hollow cylindrical portion 61 of the support member 6. In this case, the outside diameter of the hollow cylindrical portion 42 at the protuberant portions 45 is nearly equal to the inner diameter of the hollow cylindrical portion 61 of the support member 6, so that the pusher 4 is supported without chattering. In addition, the two protuberant portions 45 of the pusher 4 are each located between the two protuberant portions 64 of the support member 6.

In the condition shown in FIG. 2, the pusher 4 is locked to the support member 6 so that it would not move in the axial direction. Such locking is performed as follows. As shown in FIG. 4, projections 642 are formed to project in the circumferential direction at both ends of a base end portion of each protuberant portion 64, and base end portions of each protuberant portion 45 are locked to (in contact with) the projections 642, whereby the pusher 4 is prevented from moving in the base end direction. In addition, tip end portions of each protuberant portion 45 are locked to (in contact with) the ribs 68, whereby the pusher 4 is prevented from moving in the tip end direction.

Thus, in this embodiment, the protuberant portions 45, the protuberant portions 64 and the ribs 68 constitute a lock mechanism for locking the pusher 4 to the support member 6. With such a lock mechanism provided, the pusher 4 can be prevented from being pushed to rupture and unseal the sealing film 5 during transportation or storage of the syringe 1.

Incidentally, the lock mechanism is not limited to the configuration shown in the figures, and any configuration may be adopted, for example, engagement between recessed portions and projected portions, or projected portions and projected portions, provided respectively at the inner circumferential surface of the hollow cylindrical portion 61 and the outer circumferential surface of the hollow cylindrical portion 42, screw-engagements between screws, or the like.

The lock mechanism in this embodiment is so configured as to be unlocked by rotating the pusher 4, as will be described below. When the pusher 4 is rotated in a specified direction starting from the condition shown in FIG. 2, the protuberant portion 45 is passed between an end portion of the rib 68 and the protuberant portion 64, with an angular portion 453 on the base end side sliding on the lead surface 641 of the protuberant portion 64, and is moved to the tip end side relative to the protuberant portion 64 and the rib 68. This unlocks the pusher 4, resulting in that the pusher 4 is movable in the longitudinal direction. Incidentally, in the condition shown in FIG. 2, edge portions of the protuberant portions 45 abut on edge portions of the protuberant portions 64, whereby the pusher 4 is prevented from rotating in the direction opposite to the above-mentioned specified direction.

Thus, when the pusher 4 is rotated in the specified direction starting from the condition shown in FIG. 2, the pusher 4 is unlocked while moving in the tip end direction. Therefore, the protuberant portions 64 (the lead surfaces 641) and the protuberant portions 45 function as a leading unit for leading the pusher 4 to move in the tip end direction when the pusher 4 is rotated. With such a leading unit provided, the sealing film 5 can be ruptured and unsealed by the operation of rotating the pusher 4, as will be described later. Incidentally, the leading unit is not limited to the configuration shown in the figures. For example, the leading unit may be composed of a female screw formed in the support member 6 and a male screw formed on the pusher 4 to be screw-engaged with the female screw.

The protuberant portion 45 is provided on its outer circumferential surface with a plurality of minute ribs 452 extending in the axial direction. When the pusher 4 is rotated starting from the condition before use shown in FIG. 2, the outer circumferential surfaces of the protuberant portions 45 slide relative to the inner circumferential surface 65, with the ribs 452 climbing over the ribs 67 of the support member 6, whereby a click feeling is obtained. Besides, with the ribs 452 and the ribs 67 provided, the pusher 4 can be prevented from rotating spontaneously during transportation, storage or the like.

On the tip end side of the hollow cylindrical portion 42 is provided a connection portion (head portion) 43 for connection with the gasket 3. The connection portion 43 is provided at its outer circumference with a male screw 431 capable of screw-engagement with the female screw 331 formed at the inner surface of the hollow portion 33.

As shown in FIG. 3, after the sealing film 5 is ruptured and unsealed by the rupture portion 44 which will be described later, the connection portion 43 is inserted into the hollow portion 33 of the gasket 3 and the male screw 431 is screw-engaged with the female screw 331, whereby the gasket 3 and the pusher 4 can be connected with each other. In the connected condition, the rupture portion 44 described later is housed (stored) in the recessed portion 34 of the gasket 3.

Since the connection between the gasket 3 and the pusher 4 is thus performed through the screw-engagement structure, the connection can be performed securely, the gasket 3 and the pusher 4 are prevented from being disengaged from each other attendant on the operation on the pusher 4, and the operations to attach and detach the pusher 4 to and from the gasket 3 can be carried out easily.

Incidentally, in the present invention, the connection structure between the gasket 3 and the pusher 4 may be other than the screw-engagement; for example, a play-free mechanism such as fitting or a played mechanism such as loose fitting may be adopted.

In addition, in the present invention, a structure in which the gasket 3 and the pusher 4 are not connected with each other, specifically, a structure in which the gasket 3 is pushed to move by the pusher 4 in only the tip end direction, may also be adopted.

At the tip end portion of the pusher 4 is provided with the rupture portion 44 for rupturing the sealing film 5. In this embodiment, the rupture portion 44 is in the shape of a sharp projection roughly conical in shape, and is provided at the tip end of the connection portion 43.

In the present invention, by moving the pusher 4 in the tip end direction starting from the condition before use, the rupture portion 44 is caused to pierce the sealing film 5, whereby the sealing film 5 can be ruptured and unsealed. Therefore, an operation to peel off and remove the sealing film 5 by hand is not needed when using the syringe 1, so that an excellent operability can be obtained.

In the condition before use shown in FIG. 2, the tip end of the rupture portion 44 is preferably located in the vicinity of the base end face of the sealing film 5.

Examples of the material usable for constituting the pusher 4 include various resins such as polyvinyl chloride, polyethylene, polypropylene, polystyrene, poly-(4-methylpentene-1), polycarbonate, acrylic resin, acrylonitrile-butadiene-styrene copolymer, polyesters (e.g., polyethylene terephthalate, polyethylene naphthalate), butadiene-styrene copolymer, and polyamides (e.g., nylon 6, nylon 6 · 6, nylon 6 · 10, and nylon 12). Among these resins, preferred are such resins as polypropylene, polyesters and poly-(9-methylpentene-1), in view of easy-moldability.

Next, one example of the method of using the syringe 1 will be described. The method described below pertains to one example of the case where a double ended needled holder (not shown) comprising a bottomed tubular holder main body and a double ended needle (needle pipe) provided at its both ends with sharp needle ends is connected to the syringe 1, and a medical agent is mixingly injected into a bottle-shaped or bag-shaped injection vessel (not shown).
[1] First, the reduced diameter portion 22 of the syringe 1 before use shown in FIG. 2 is connected to a mouth portion of the injection vessel through the double ended needled holder. Specifically, one needle end of the double ended needled holder is made to pierce through a plug body sealing the mouth portion of the injection vessel, and the other needle end is made to pierce through the film 24. Upon this connection, an injection in the injection vessel is sucked and led into the outer tube 2 through the double ended needle, since the inside of the outer tube 2 is preliminarily set in a decompressed state.
[2] Next, the pusher 4 is rotated in a specified direction. Upon the rotation of the pusher 4, the pusher 4 is unlocked from the support member 6 while moving in the tip end direction being led by the lead surface 641. In addition, simultaneously with unlocking, the movement of the pusher 4 in the tip end direction causes the rupture portion 44 to pierce the sealing film 5, whereby the sealing film 5 is ruptured and unsealed.
[3] When the pusher 4 is further rotated in the same direction while being advanced in the tip end direction, the connection portion 43 is inserted into the hollow portion 33 of the gasket 3, and the male screw 431 and the female screw 331 are screw-engaged with each other, whereby the gasket 3 and the pusher 4 are connected with each other (see FIG. 3).
   Incidentally, when the hollow cylindrical portion 42 passes through the vicinity of the base end opening of the outer tube 2, the ruptured sealing film 5 is inserted into the relief portion 29, and therefore, does not hamper the passage.
   In addition, where the inside of the outer tube 2 is not preliminarily set in the decompressed state, the pusher 4 in this instance is pushed to slide the gasket 3 in the outer tube 2 in the tip end direction and then the pusher 4 is pulled to slide the gasket 3 in the base end direction, whereby the injection is sucked and led in.
[4] The syringe 1 is oscillated or vibrated, and the medical agent 100 is thereby dissolved or dispersed in the injection led into the outer tube 2, to obtain a medical agent containing the medical agent 100 as the effective constituent or constituents. Or, the syringe 1 may be oscillated or vibrated after the syringe 1 is once detached from the double ended needled holder, if necessary.
[5] The pusher 4 is pushed in the tip end direction. This causes the gasket 3 to slide in the outer tube 2 in the tip end direction, whereby the chemical in the outer tube 2 is discharged through the double ended needle, to be blended into the injection contained in the injection vessel.

As is seen from the operations described in the above paragraphs [1] to [5], the syringe 1 according to the present invention does not need an operation to peel off and remove the sealing film 5. Therefore, the operations can be performed easily and speedily.

In addition, since the pusher 4 is supported by the support member 6 and combined with the outer tube 2 before use, there is no possibility that the pusher 4 might be lost and, therefore, it is unnecessary to search for the pusher 4 before use.

Besides, in this embodiment, since the pusher 4 is locked to the support member 6 before use, the pusher 4 is prevented from spontaneously moving in the tip end direction during transportation or storage, and therefore, the rupture portion 44 can be prevented from rupturing the sealing film 5 before use. Particularly, in this embodiment, since the pusher 4 is unlocked by rotating it, the pusher 4 would not be unlocked even if the finger receiving portion 411 is pushed and a force (impact) for moving the pusher 4 in the tip end direction is exerted during transportation or storage. Therefore, rupture of the sealing film 5 before use can be prevented more securely.

In addition, in this embodiment, the operation to rotate the pusher 4 moves the pusher 4 in the tip end direction, and the movement can cause the rupture portion 44 to rupture the sealing film 5. Therefore, the sealing film 5 can be securely ruptured by a comparatively small operating force, and the sealing film 5 can be unsealed more easily.

Besides, in this embodiment, a further rotation of the pusher 4 in the same direction after the rupture of the sealing film 5 connects the gasket 3 and the pusher 4 with each other. Since the gasket 3 and the pusher 4 can thus be connected with each other by the operation in the same direction as the operation for rupturing the sealing film 5, the rupture of the sealing film 5 and the connection between the gasket 3 and the pusher 4 can be performed in a stroke by a series of actions (continuous actions), resulting particularly in an excellent operability.

Incidentally, the method of using the syringe 1 is not limited to the above-mentioned method in which the double ended needled holder is used. It is natural that the syringe 1 can be used by fitting or mounting, for example, a hub, connectors, tubes or the like (not shown) of a needle pipe to the reduced diameter portion 22.

### <Second Embodiment>

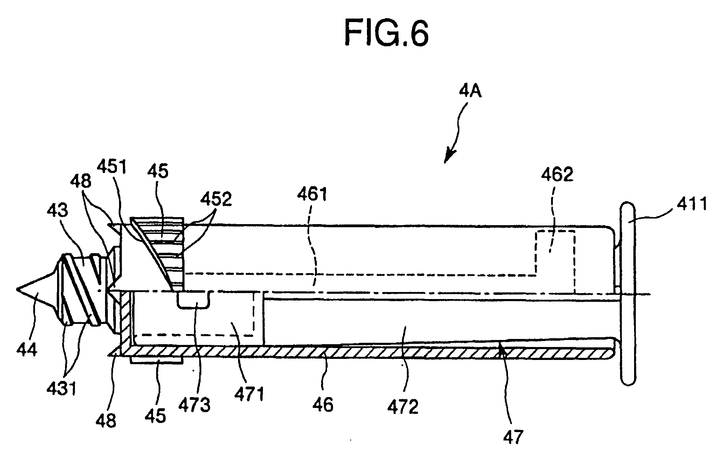

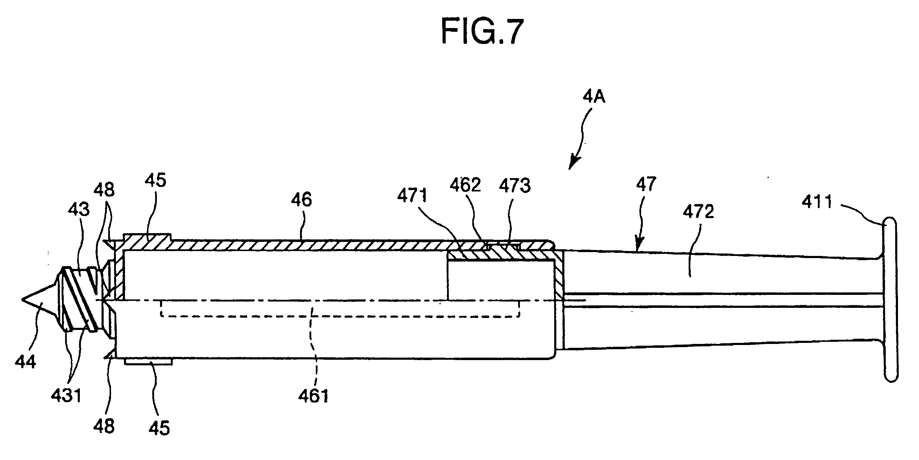

FIGS. 6 and 7 are partly vertical sectional views showing a pusher in a second embodiment of the syringe according to the present invention.

Now, the second embodiment of the syringe according to the present invention will be described below referring to these figures. In the following, description will be centered on differences from the above-described embodiment, and descriptions of the same items as above will be omitted.

This embodiment is the same as the first embodiment above, except for the differences in the configuration of the pusher.

The pusher 4A in this embodiment has an extension-contraction mechanism capable of extending and contracting the whole length thereof.

The pusher 4A comprises a bottomed tubular first member 46, and a second member 47 inserted into the first member 46 through the base end opening of the first member 46 and disposed to be movable in the longitudinal direction.

On the tip end side of the first member 46 are provided a connection portion 43 and a rupture portion 44, which are the same as above-described. Protuberant portions 45 of the same as above-described are formed at the outer circumferential surface of a tip end portion of the first member 46.

The inner circumferential surface of the first member 46 is provided with a groove 461 extending in the longitudinal direction and with a groove 462 extending in the circumferential direction from a base end portion of the groove 461. Namely, the groove 461 and the groove 462 are formed to be substantially L-shaped.

The second member 47 comprises a slide portion 471 having a generally circular tubular shape and slid relative to the inner circumferential surface of the first member 46, and a main body portion 472 provided on the base end side of the slide portion 471 and having a shape such that plate pieces intersect in a cross form. The outside diameter of the slide portion 471 is nearly equal to or slightly smaller than the inside diameter of the first member 46. At the base end of the main body portion 472 is provided a finger receiving portion 411, which is the same as above-described.

A projected portion 473 is formed on the outer circumferential surface of the slide portion 471. The projected portion 473 is inserted in the groove 461 or the groove 462 so as to be movable along the groove 461 or 462.

As shown in FIG. 6, most part on the base end side of the second member 47 can be inserted (contained) in the first member 46, whereby the pusher 4A can be set in the state where the whole length thereof is contracted. In this state, the projected portion 473 is located at a tip end portion of the groove 461.

In order to put the pusher 4A into an extended state, the second member 47 is pulled and moved in the base end direction starting from the condition shown in FIG. 6, and when the projected portion 473 reaches the base end portion of the groove 461, the second member 47 is rotated, whereby the projected portion 473 is moved along the groove 462. As a result, the pusher 4A is put into the extended state where the main body portion 472 is projected from the base end of the first member 46, as shown in FIG. 7. In this state, the projected portion 473 is engaged with the groove 462, whereby the pusher 4A is prevented from contracting. Besides, once the second member 47 is rotated, the projected portion 473 rides over a projection (not shown) formed on the inner surface of the groove 462, whereby the second member 47 is locked so that it cannot be rotated in the opposite direction relative to the first member 46, and the pusher 4A is therefore prevented from returning to the contracted state.

In this embodiment, the pusher 4A is set in the contracted state shown in FIG. 6 before use (the condition corresponding to FIG. 2), and the pusher 4A is extended as above-described when using. This makes it possible to reduce the whole length of the syringe 1 before use and to reduce the space in the syringe 1 during transportation and storage.

Besides, in the pusher 4A according to this embodiment, rupture aid portions 48 for cutting the sealing film 5 in the vicinity of an edge portion of the base end opening of the outer tube 2 are provided. The rupture aid portions 48 have a triangular blade-like shape, and are formed on an outer circumferential portion of the tip end of the first member 46. In the configuration shown, a plurality of (four) rupture aid portions 48 are provided, and are disposed at equal angular intervals (of 90°) along the circumferential direction.

The provision of such rupture aid portions 48 ensures that, when the pusher 4A is rotated starting from the condition before use, the rupture portion 4 ruptures the sealing film 5 and, simultaneously, the rupture aid portions 48 are rotated along an edge portion of the base end opening of the outer tube 2 to cut the sealing film 5. The sealing film 5 thus cut is in the state of being clamped between the base end face 35 of the gasket 3 and the tip end face of the first member 46. Therefore, the ruptured sealing film 5 can be prevented from remaining at the base end opening of the outer tube 2.

### <Third Embodiment>

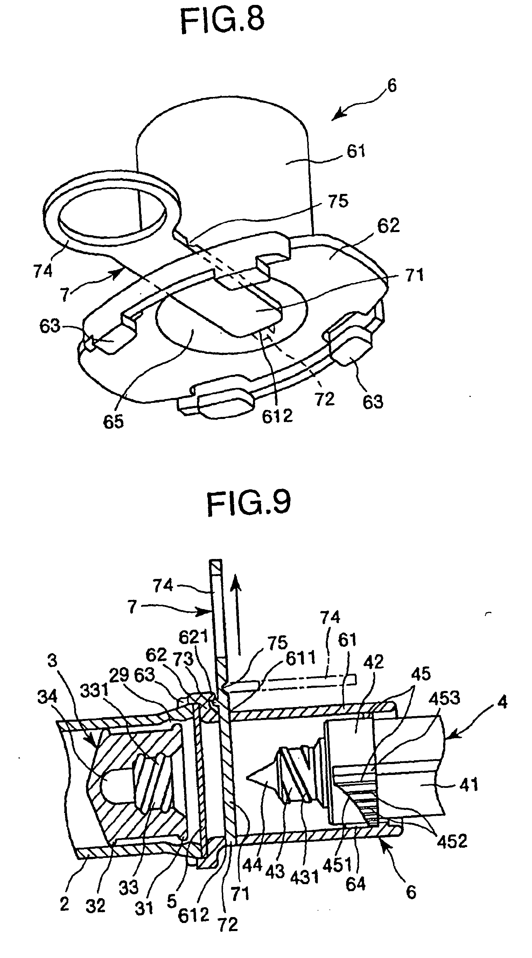

FIG. 8 is a perspective view of a support member in a third embodiment of the syringe according to the present invention, and FIG. 9 is a vertical sectional view of the third embodiment of the syringe according to the present invention in the vicinity of the support member.

Now, the third embodiment of the syringe according to the present invention will be described below referring to these figures. In the following, descriptions will be centered on differences from the above-described embodiments, and descriptions of the same items as above will be omitted.

This embodiment is the same as the first embodiment above, except that a movement preventive member 7 for preventing a pusher 4 from moving in the tip end direction is removably provided on the base end side of a sealing film 5.

As shown in FIG. 8, the movement preventive member 7 comprises a plate-like movement preventive plate 71. As shown in FIG. 9, a hollow cylindrical portion 61 of a support member 6 is provided in its tip end portion with a hole (slit) 611 in which the movement preventive plate 71 is inserted. The support member 6 is provided with the movement preventive member 7 in the condition where the movement preventive plate 71 is inserted in the hollow cylindrical portion 61 via the hole 611.

In this condition, the movement preventive plate 71 is located between the sealing film 5 and a rupture portion 44. Therefore, even if a force for moving the pusher 4 in the tip end direction is exerted, the rupture portion 44 abuts on the base end face of the movement preventive plate 71, whereby the pusher 4 can be prevented from moving in the tip end direction. This ensures that the sealing film 5 can be more securely prevented from being ruptured and unsealed during transportation or storage of the syringe 1. Besides, even if the pusher 4 is erroneously operated, the sealing film 5 can be securely prevented from being ruptured.

As shown in FIG. 8, the movement preventive plate 71 is provided with a projected portion 72 at a lower end portion in the figure, and the projected portion 72 is inserted in a hole 612 formed in the hollow cylindrical portion 61. This ensures that the movement preventive member 7 is supported at two locations, i.e., at the holes 611 and 612, and is disposed on the support member 6 without generation of chattering or the like.

In addition, as shown in FIG. 9, a projected portion 73 is formed on the tip end face of the movement preventive plate 71 in the vicinity of the outside of the hole 611. The projected portion 73 is engaged to a projection portion 621 formed on the base end face of a flange 62, whereby the movement preventive member 7 is prevented from being disengaged from the support member 6.

The movement preventive plate 71 is provided with a ring-shaped handle 74 on the upper side in FIG. 9. At the time of removing the movement preventive member 7, a finger is engaged with the handle 74, and the movement preventive member 7 is pulled to the upper side in Fig. 9, upon which the projected portion 73 rides over the projected portion 621 to cause disengagement of them from each other, resulting in that the movement preventive plate 71 can be pulled out of the hole 611. When using the syringe 1 according to this embodiment, the movement preventive member 7 is thus removed, and then the operations described in the above paragraphs [1] to [5] are carried out.

The movement preventive plate 71 is provided, on the lower side of the handle 74 in FIG. 9, with a thinned portion 75 in the manner of crossing the movement preventive plate 71, so that the movement preventive plate 71 can be bent at the thinned portion 75. This ensures that the handle 74 can be folded as indicated by dot-dash lines in FIG. 9 during transportation or storage, so that the handle 74 is not bulky, and the space therefore can be reduced.

While the syringe according to the present invention has been described above referring to the embodiments thereof shown in the figures, the present invention is not limited to the embodiments. Components of the syringe, particularly, the structure of the pusher, the structure of the gasket, the structure of the support member, and the like are not limited to the configurations shown in the figures, and may be replaced by arbitrary ones capable of displaying the functions equivalent to the above-described.

In addition, the syringe according to the present invention may be composed of a combination of two or more configurations (characteristic features) selected from the above-described embodiments.

As has been described above, according to the present invention, the need for an operation to peel off and remove the sealing film for sealing the base end opening of the outer tube is eliminated. Therefore, the operations to use the syringe can be performed easily and speedily.

Besides, since the pusher is supported by the support member and combined with the outer tube before use, the pusher is prevented from being lost, and it is unnecessary to search for the pusher before use.

In addition, where the lock mechanism for locking the pusher to the support member before use and the movement preventive member for preventing the pusher from moving in the tip end direction are provided, the sealing film can be prevented from being erroneously ruptured and unsealed by the rupture portion during transportation or storage.

## Claims

1. A syringe comprising:
an outer tube;
a gasket slidable in said outer tube;
a sealing film for sealing the base end opening of said outer tube;
a pusher for operating said gasket to move in the longitudinal direction of said outer tube, said pusher being provided at a tip end portion thereof with a rupture portion for rupturing said sealing film; and
a support member for supporting said pusher generally coaxially with said outer tube on the base end side of said sealing film; wherein
said pusher is supported by said support member before use and, at the time of use, said pusher is moved in the tip end direction relative to said outer tube, thereby causing said rupture portion to rupture and unseal said sealing film.

2. A syringe as set forth in claim 1, wherein said rupture portion is in the shape of a sharp projection.

3. A syringe as set forth in claim 1 or 2, wherein said pusher comprises a connection portion for connection with said gasket so that said pusher is connectable with said gasket.

4. A syringe as set forth in any of claims 1 to 3, wherein the inside of said outer tube is in a decompressed state before use.

5. A syringe as set forth in any of claims 1 to 4, wherein the inside of said outer tube is in a sterilized state before use.

6. A syringe as set forth in any of claims 1 to 5, wherein a medical agent is preliminarily contained in the space defined by said outer tube and said gasket.

7. A syringe as set forth in any of claims 1 to 6, wherein said pusher comprises an extension-contraction mechanism for making the whole length thereof extensible and contractible.

8. A syringe as set forth in any of claims 1 to 7, wherein a movement preventive member for preventing said pusher from moving in the tip end direction is removably provided on the base end side of said sealing film.
